# EUROPEAN PATENT SPECIFICATION

(11) **EP 0 504 239 B1**
(45) Date of publication and mention of the grant of the patent: **13.05.1998**
(21) Application number: 91900862.3
(22) Date of filing: 06.12.1990
(51) Int. Cl.: A61K 38/46

(54) **ANISTREPLASE FOR TREATING STROKE**
ANISTREPLASE ZUR BEHANDLUNG DES HIRNSCHLAGS
ANISTREPLASE POUR LE TRAITEMENT D'INFARCTUS

(30) Priority: 13.12.1989 GB 8928163
(43) Date of publication of application: 23.09.1992
(73) Proprietor: ROBERTS LABORATORIES INC., Eatontown, NJ 07724 (US)
(72) Inventor: FEARS, Robin, Bradshaw SmithKline Beecham Pharmac, Epsom Surrrey KT18 5XQ (GB); DUFF, David, Alexander, Reigate RH2 9HF (GB)
(74) Representative: Mallalieu, Catherine Louise
(86) International application number: GB9001905
(87) International publication number: WO9108768

(56) References cited:
- EP-A- 0 028 489
- Stroke, vol. 17, no. 4, 1986; G.J. Del Zoppo et al.: "Thrombolytic therapy in stroke: possibilities and hazards", pages 595-607
- Drugs, vol. 33 (suppl. 3), 1987, ADIS Press Ltd; M.L. Brochier et al.: "Intravenous anisoylated plasminogen streptokinase activator complex versus intravenous streptokinase in evolving myocardial infaction", pages 140-145
- Drugs, vol. 34, 1987, ADIS Press Ltd; J.P. Monk et al.: "Anisoylated plaminogen streptokinase activator complex (APSAC)", pp. 25-49

## Description

The present invention relates to a method for the treatment of stroke and to the use of a compound in the preparation of a medicament for the treatment of stroke.

In Stroke 17, 535-607 (1986) stroke therapy by intravenous administration of a streptokinase-plasminogen complex (Fibrinolysin®) is disclosed.

EP-0028489 discloses p-anisoyl streptokinase lys-human plasminogen activator complex, British Approved name anistreplase, and a process by which it can be prepared.

Anistreplase is described in EP-0028489 as a thrombolytic agent and is used clinically in the treatment of acute myocardial infarction.

The present invention provides use of anistreplase in the manufacture of a medicament for the treatment of stroke in mammals including humans. Preferably the mammal is a human.

The administration is preferably by way of a pharmaceutical composition adapted for intravenous administration to human beings.

Typically compositions for intravenous administration are solutions of the sterile complex in sterile aqueous buffer. Where necessary the composition may also include a solubilising agent to keep the complex in solution and a local anaesthetic such as lignocaine to ease pain at the site of injection. Generally, the complex will be supplied in unit dosage form for example as a dry powder or water free concentrate in a hermetically sealed container such as a vial, ampoule or sachette indicating the quantity of complex in activity units. Since the complex includes a removable blocking group, an indication of the time within which the free complex will be liberated may be given. Where the complex is to be administered by infusion, it will be dispensed with an infusion bottle containing sterile pharmaceutical grade 'Water for Injection' or saline. Where the complex is to be administered by injection, it is dispensed with an ampoule of sterile water for injection or saline. The injectable or infusable composition will be made up by mixing the ingredients prior to administration.

The quantity of material administered will depend upon the amount of fibrinolysis required and the speed with which it is required, the seriousness of the thromboembolic condition and position and size of the clot. The precise dose to be employed and mode of administration must in view of the nature of the complaint be decided according to the circumstances by the physician supervising treatment. However, in general, a patient being treated for a thrombus will generally receive a total dose of from 0.01 to 10 mg/kg of body weight, such as 0.10 to 2.0mg/kg, preferably by injection in for example up to five doses, most preferably one dose, or alternatively by infusion.

Such preparation and treatment may be carried out in the manner as described hereinbefore.

The present invention uses a pharmaceutical composition in the treatment of stroke in mammals including humans, which comprises an effective amount of anistreplase and a pharmaceutical carrier. Such composition may be prepared in the manner as described hereinbefore.

### Clinical Test

Patients are treated within 3 hours of the symptom onset of ischaemic stroke with 10-30 U anistreplase administered intravenously over 2 to 5 minutes. An assessment of neurological function is made pretreatment and at intervals thereafter to determine efficacy.

## Claims

1. Use of anistreplase in the manufacture of a medicament for the treatment of stroke in mammals including humans.

2. Use according to claim 1 wherein the mammal is a human.

3. Use according to claim 1 or claim 2 wherein the medicament is adapted for intravenous administration.

## Patentansprüche

1. Verwendung von Anistreplase bei der Herstellung eines Arzneimittels zur Behandlung von Gehirnschlag bei Säugetieren einschließlich Menschen.

2. Verwendung nach Anspruch 1, bei der das Säugetier ein Mensch ist.

3. Verwendung nach Anspruch 1 oder Anspruch 2, bei der das Arzneimittel für intravenöse Verabreichung geeignet ist.

## Revendications

1. Utilisation d'anistreplase dans la production d'un médicament pour le traitement d'ictus chez des mammifères, y compris l'homme.

2. Utilisation suivant la revendication 1, dans laquelle le mammifère est un patient humain.

3. Utilisation suivant la revendication 1 ou la revendication 2, dans laquelle le médicament est apte à l'administration intraveineuse.
